(19) **Europäisches Patentamt / European Patent Office / Office européen des brevets**

(11) **EP 2 626 009 A1**

(12) # EUROPEAN PATENT APPLICATION
published in accordance with Art. 153(4) EPC

(43) Date of publication:
14.08.2013 Bulletin 2013/33

(21) Application number: 11830367.6

(22) Date of filing: 03.10.2011

(51) Int Cl.:
*A61B 8/08* (2006.01)   *A61B 8/06* (2006.01)

(86) International application number:
PCT/JP2011/005568

(87) International publication number:
WO 2012/046433 (12.04.2012 Gazette 2012/15)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 08.10.2010 JP 2010228284

(71) Applicant: Panasonic Corporation
Osaka 571-8501 (JP)

(72) Inventor: TOJI, Bunpei
Osaka-shi
Osaka 540-6207 (JP)

(74) Representative: Vigand, Philippe et al
Novagraaf International SA
Chemin de l'Echo 3
1213 Onex (CH)

(54) **ULTRASONIC DIAGNOSTIC DEVICE AND ULTRASONIC DIAGNOSTIC METHOD**

(57) An ultrasound diagnostic apparatus (150) which observes an inside of a body of a subject based on reflected ultrasound waves (201) reflected from the inside of the body and obtained using an ultrasound probe (101), includes: a B-mode image generation unit (104) which generates a B-mode image (202), based on the reflected ultrasound waves (201); a blood flow image generation unit (105) which generates, based on the reflected ultrasound waves (201), a blood flow image (203) representing a region of the body in which blood flows; a lumen contour extraction unit (106) which extracts a lumen contour (402) of a blood vessel, based on the blood flow image (203); a provisional adventitia contour setting unit (107) which sets a provisional adventitia contour (407) containing the lumen contour (402); and an adventitia contour extraction unit (108) which extracts an adventitia contour (401) of the blood vessel, using the B-mode image (202), and using the provisional adventitia contour (407) as an initial contour.

FIG. 1

**Description**

[Technical Field]

The present invention relates to ultrasound diagnostic apparatuses and ultrasound diagnostic methods, and particularly relates to an ultrasound diagnostic apparatus which observes an inside of a body of a subject based on ultrasound waves reflected from the inside of the body and obtained using an ultrasound probe.

[Background Art]

In recent years, ultrasound diagnostic apparatuses are being used for early discovery of arteriosclerosis, vascular diseases, and so on. Specifically, ultrasound diagnostic apparatuses are being used to measure intima-media thickness (hereafter called IMT) which is the combined membrane thickness of the intima and media of blood vessel walls. Furthermore, ultrasound diagnostic apparatuses are being used to verify the presence of plaque formed due to the narrowing of the lumen of a blood vessel. This is because it has become clear that IMT increases and plaque is formed as arteriosclerosis advances. Moreover, it is considered that arteriosclerosis advances throughout the entire body, and thus superficial carotid arteries become the main target for measurement in measuring IMT and judging the presence of plaque. Here, plaque refers to an elevated lesion which is a localized projection of the inner wall of the blood vessel towards the inner side (lumen) of the blood vessel. Plaque assumes various forms such as thrombus, fatty, fibrous, and so on, and can be a cause for the narrowing and occlusion of carotid arteries as well as cerebral infarcts and cerebral ischemia.

Detecting the shape of plaque using an ultrasound diagnostic apparatus is performed using ultrasound images of blood vessels. The ultrasound diagnostic apparatus sends ultrasound waves to the inside of the body of a subject via a probe, and forms an ultrasound image (for example, a B-mode image) based on reflected waves created from the subject. Then, the examiner judges the presence of plaque by looking at the ultrasound image.

Here, when the examiner judges the presence of plaque, the examiner manually specifies (sketches) the shape of the blood vessel adventitia and blood vessel intima in the ultrasound image, and performs the diagnosis based on this sketch. Specifically, the examiner sketches, on the B-mode image, the shape of the periphery of the adventitia as an adventitia contour line, and further sketches the shape of the periphery of the lumen as the lumen contour line. Then, at the end, the examiner performs the diagnosis for the presence of plaque, and so on, based on the shape of the sketch (see for example, Non Patent Literature (NPL) 1.

However, in the method in NPL 1, after obtaining the image, it is necessary to manually specify the positions of the adventitia contour and the intima contour of the blood vessel wall on the ultrasound image in an on-line state. Specifically, performing plaque detection using this conventional diagnostic method requires manual contour specification and is thus troublesome for the examiner. As a result, there are problems such as the examination time becoming long and fluctuation occurring in the results of the examination by the examiner.

In response to these problems, NPL 2 and Patent Literature (PTL) 1 describe a method of automatically extracting a blood vessel wall contour by applying active contour searching to a B-mode image which is an ultrasound diagnostic image. With this method, it becomes possible to reduce the burden on the examiner by reducing the trouble of manual tasks and to shorten the examination time.

[Citation List]

[Patent Literature]

[PTL 1] Japanese Patent No. 3468869

[Non Patent Literature]

[NPL 1] Ainsworth CD, Blake CC, Tamayo A, Beletsky V, Fenster A, Spence JD, "3D ultrasound measurement of change in carotid plaque volume: a tool for rapid evaluation of new therapies", Stroke 2005 Sep; 36 (9): 1904-1909
[NPL 2] J.C.R. Seabra, L.M. Pedro, J.F. e Fernandes, and J.M. Sanches, "A 3-D Ultrasound based Framework to Characterize the Echo Morphology of Carotid Plaques", IEEE Trans. Biomed. Eng., vol. 56, pp. 1442 2009

[Summary of Invention]

[Technical Problem]

However, in the diagnostic methods in PTL 1 and NPL 2, there are cases where the contour of the blood vessel wall cannot be extracted accurately even when attempting to correctly extract the contour of the blood vessel wall using an ultrasound diagnostic apparatus. For example, when the contour (border) of the object is positioned parallel or is in a state that is close to being parallel to the ultrasound waves transmitted from ultrasound transducers, there are cases where the border of the contour line is not clearly displayed on the ultrasound image. As such, when an image that is perpendicular to the running direction of the blood vessel is generated, there is a tendency for it to be difficult to clearly display the lateral contour of the blood vessel wall compared to the upper and lower contour of the blood vessel wall. This tendency can be seen when displaying either of the lumen contour or the adventitia contour of the blood vessel. As such, it was not possible to accurately trace the contour of the blood vessel wall using the conventional method of searching for the contour based on the data of an ultrasound image.

The present invention solves the aforementioned conventional problems and has as an object to provide an ultrasound diagnostic apparatus capable of more accurately extracting the contour of a blood vessel wall.

[Solution to Problem]

In order to solve the conventional problems, the ultrasound diagnostic apparatus according to an aspect of the present invention is an ultrasound diagnostic apparatus which observes an inside of a body of a subject based on ultrasound waves reflected from the inside of the body and obtained using an ultrasound probe, the ultrasound diagnostic apparatus including: a B-mode image generation unit configured to generate a B-mode image, based on the reflected ultrasound waves; a blood flow information generation unit configured to generate, based on the reflected ultrasound waves, blood flow information representing a region of the body in which blood flows; a lumen contour extraction unit configured to extract a lumen contour of a blood vessel, based on the blood flow information; a provisional adventitia contour setting unit configured to set a provisional adventitia contour containing the lumen contour; and an adventitia contour extraction unit configured to extract an adventitia contour of the blood vessel, using the B-mode image, and using the provisional adventitia contour as a first initial contour.

According to this configuration, the ultrasound diagnostic apparatus according to an aspect of the present invention extracts a lumen contour based on the blood flow information. With this, the ultrasound diagnostic apparatus can extract the lumen contour more accurately, compared to when the lumen contour is extracted using a B-mode image. In addition, the ultrasound diagnostic apparatus sets a provisional adventitia contour using the lumen contour extracted based on the blood flow information, and extracts an adventitia contour using such provisional adventitia contour. With this, the ultrasound diagnostic apparatus can extract the adventitia contour more accurately. In this manner, the ultrasound diagnostic apparatus can extract the contour of the blood vessel wall more accurately.

Furthermore, the ultrasound diagnostic apparatus according to an aspect of the present invention may further include: a blood vessel wall extraction unit configured to extract, using the lumen contour and the adventitia contour, a blood vessel wall region in which a blood vessel wall is present; and a plaque region extraction unit configured to extract, using the blood vessel wall region, a plaque region in which plaque is present.

According to this configuration, the ultrasound diagnostic apparatus according to an aspect of the present invention can extract the plaque region more accurately.

Furthermore, the ultrasound diagnostic apparatus according to an aspect of the present invention may further include: a three-dimensional data generation unit configured to generate three-dimensional data representing the blood vessel wall region and the plaque region; and a display unit configured to display the three-dimensional data.

According to this configuration, the ultrasound diagnostic apparatus according to an aspect of the present invention can display the plaque region using three-dimensional data.

Furthermore, the provisional adventitia contour setting unit may be configured to set the provisional adventitia contour having a circular shape.

According to this configuration, the ultrasound diagnostic apparatus according to an aspect of the present invention can set a provisional adventitia contour that is close to the shape of the adventitia contour.

Furthermore, the provisional adventitia contour setting unit may be configured to: set a circle having a center point of the lumen contour as a center and a radius that is greater than a maximum distance from the center point to the lumen contour, and perform, on the blood flow information and using the set circle as a second initial contour, first active contour searching to search for a contour such that a sum of an inner deformation energy of a contour line and an image energy is smallest, and set the contour obtained through the search as the provisional adventitia contour, the image energy representing conformity between the contour line and an image.

According to this configuration, the ultrasound diagnostic apparatus according to an aspect of the present invention can set a provisional adventitia contour that is close to the shape of the adventitia contour.

Furthermore, the provisional adventitia contour setting unit may be configured to perform convergence processing by making a weight of the image energy smaller than a weight of the inner deformation energy in the first active contour searching.

According to this configuration, the ultrasound diagnostic apparatus according to an aspect of the present invention can carry out a form constraint such that the contour line does not become convex inward.

Furthermore, the adventitia contour extraction unit may be configured to perform, on the B-mode image and using the provisional adventitia contour as the first initial contour, second active contour searching to search for a contour such that a sum of an inner deformation energy of a contour line and an image energy is smallest, and set the contour obtained through the search as the provisional adventitia contour, the image energy representing conformity between the contour line and an image.

According to this configuration, the ultrasound diagnostic apparatus according to an aspect of the present invention can extract the adventitia contour more accurately.

Furthermore, the provisional adventitia contour setting unit may be configured to set a weight of the image energy with respect to the inner deformation energy to a second value when clarity of the B-mode image is a first value, and to set the weight to a fourth value which is larger than the second value when the clarity is a third value larger than the first value, in the second active contour searching.

According to this configuration, the ultrasound diagnostic apparatus according to an aspect of the present invention can extract the adventitia contour precisely, in accordance with the state of depiction of the blood vessel contour.

Furthermore, the provisional adventitia contour setting unit may be configured to change the weight in accordance with the clarity, for each of contour points in the B-mode image.

According to this configuration, the ultrasound diagnostic apparatus according to an aspect of the present invention can extract the adventitia contour more precisely.

Furthermore, the blood flow information generation unit may be configured to generate the blood flow information, using a color Doppler method.

Furthermore, the provisional adventitia extraction unit may include an initial contour position adjustment unit, and the initial contour position adjustment unit may be configured to: calculate a center point of a blood vessel region, using the B-mode image; and move the provisional adventitia contour so that a center point of the provisional adventitia contour approaches the center point of the blood vessel region.

According to this configuration, the ultrasound diagnostic apparatus according to an aspect of the present invention can stably extract a correct adventitia contour even when there is a misalignment of images between the B-mode image and the blood flow information.

It should be noted that the present invention can be implemented, not only as an ultrasound diagnostic apparatus such as that described herein, but also as an ultrasound diagnostic method, a blood vessel contour extraction method, a blood vessel wall extraction method, or a plaque region extraction method having, as steps, the characteristic processing units included in the ultrasound diagnostic apparatus, and also as a program which causes a computer to execute such characteristic steps. In addition, it goes without saying that such a program can be distributed via a non-transitory computer-readable recording medium such as a CD-ROM and via a transmitting medium such as the Internet.

Moreover, the present invention can be implemented as a semiconductor integrated circuit (LSI) in which part or all of the function of such an ultrasound diagnostic apparatus are implemented, or as an ultrasound diagnostic system including such an ultrasound diagnostic apparatus.

[Advantageous Effects of Invention]

As described above, the present invention can provide an ultrasound diagnostic apparatus capable of more accurately extracting the contour of a blood vessel wall.

[Brief Description of Drawings]

[FIG. 1] FIG. 1 is a block diagram of an ultrasound diagnostic apparatus according to Embodiment 1 of the present invention.
[FIG. 2] FIG. 2 is a block diagram of the ultrasound diagnostic apparatus according to Embodiment 1 of the present invention.
[FIG. 3] FIG. 3 is a cross-sectional view of a blood vessel according to Embodiment 1 of the present invention.
[FIG. 4] FIG. 4 is a flowchart of a blood vessel wall extraction process performed by the ultrasound diagnostic apparatus according to Embodiment 1 of the present invention.

[FIG. 5A] FIG. 5A is an image illustrating a B-mode image of the blood vessel, according to Embodiment 1 of the present invention.

[FIG. 5B] FIG. 5B is an image illustrating a contour displayed in the B-mode image, according to Embodiment 1 of the present invention.

[FIG. 6A] FIG. 6A is a diagram showing a result of an active contour search when a normal energy function is set, according to Embodiment 1 of the present invention.

[FIG. 6B] FIG. 6B is a diagram showing a result of an active contour search when an function for emphasizing outward searching is set, according to Embodiment 1 of the present invention.

[FIG. 7] FIG. 7 is a diagram showing a high brightness region (noise) inside the blood vessel wall, according to Embodiment 1 of the present invention.

[FIG. 8] FIG. 8 is a diagram showing provisional adventitia contour extraction processing according to Embodiment 1 of the present invention.

[FIG. 9] FIG. 9 is a diagram showing an extracted provisional adventitia according to Embodiment 1 of the present invention.

[FIG. 10] FIG. 10 is a flowchart of a contour extraction process performed by the ultrasound diagnostic apparatus according to Embodiment 1 of the present invention.

[FIG. 11] FIG. 11 is a block diagram of an ultrasound diagnostic apparatus according to a modification of Embodiment 1 of the present invention.

[FIG. 12A] FIG. 12A is a diagram showing three-dimensional data generation processing according to the modification of Embodiment 1 of the present invention.

[FIG. 12B] FIG. 12B is a diagram showing three-dimensional data generation processing according to a modification of Embodiment 1 of the present invention.

[FIG. 13] FIG. 13 is a block diagram of an ultrasound diagnostic apparatus according to Embodiment 2 of the present invention.

[Description of Embodiments]

Hereinafter, embodiments of the present invention shall be described with reference to the Drawings. It should be noted that the embodiments described hereafter illustrate preferred specific examples of the present invention. Numerical values, shapes, materials, constituent elements, the positioning and connection configuration of the constituent elements, steps, the sequence of the steps, and so on, described in the embodiments below are merely examples and are not intended to limit the present invention. The present invention is only limited by the Claims. Therefore, among the constituent elements in the following embodiments, those constituent elements which are not described in the independent claims indicating the broadest concept of the present invention are not necessarily required in order to achieve the object of the present invention but are described as elements for configuring a more preferable embodiment.

[Embodiment 1]

An ultrasound diagnostic apparatus according to Embodiment 1 of the present invention extracts a lumen contour of a blood vessel based on a blood flow image, and sets a provisional adventitia contour containing the extracted lumen contour. In addition, the ultrasound diagnostic apparatus uses the set provisional adventitia contour as an initial contour, and extracts an adventitia contour of the blood vessel using a B-mode image.

With this, the ultrasound diagnostic apparatus according to Embodiment 1 of the present invention is able to more accurately extract the contour of the blood vessel wall.

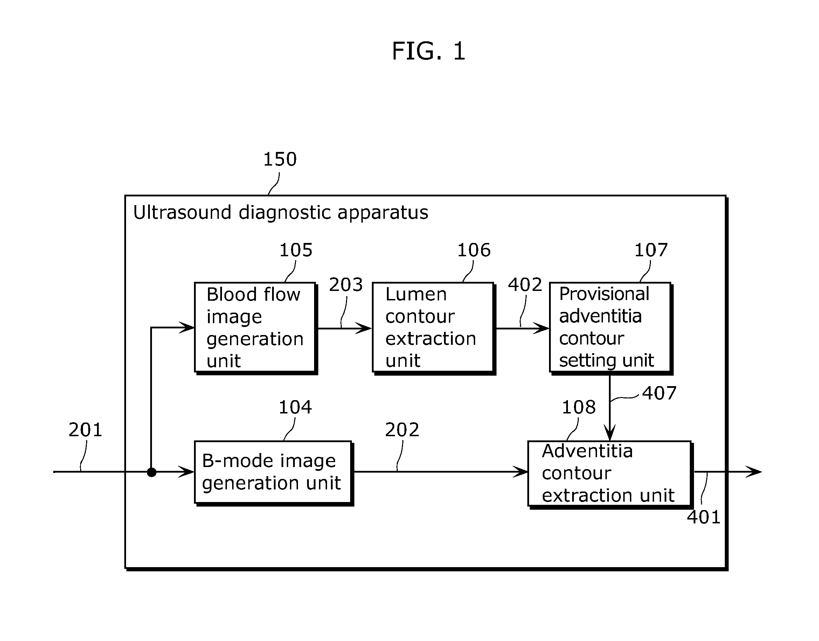

First, an outline configuration of the ultrasound diagnostic apparatus according to Embodiment 1 of the present invention shall be described. FIG. 1 is a block diagram showing the outline configuration of the ultrasound diagnostic apparatus according to Embodiment 1 of the present invention.

An ultrasound diagnostic apparatus 150 shown in FIG. 1 observes the inside of a body of a subject, based on reflected ultrasound waves 201 which are ultrasound waves reflected from the inside of the body which are obtained using an ultrasound probe. The ultrasound diagnostic apparatus 150 includes a B-mode image generation unit 104, a blood flow image generation unit 105, a lumen contour extraction unit 106, a provisional adventitia contour setting unit 107, and an adventitia contour extraction unit 108.

The B-mode image generation unit 104 generates a B-mode image 202 based on the reflected ultrasound waves 201.

The blood flow image generation unit 105 corresponds to the blood flow information generation unit of the present invention. The blood flow image generation unit 105 generates a blood flow image 203 showing a region in which blood flows, based on the reflected ultrasound waves 201. Here, the blood flow image 203 corresponds to the blood flow information of the present invention.

The lumen contour extraction unit 106 extracts a lumen contour 402 of the blood vessel based on the blood flow image 203.

The provisional adventitia contour setting unit 107 sets a provisional adventitia contour 407 containing the lumen contour 402.

With the provisional adventitia contour 407 as an initial contour, the adventitia contour extraction unit 108 extracts an adventitia contour 401 of the blood vessel using a B-mode image 202.

Details of the configuration of the ultrasound diagnostic apparatus 150 according to Embodiment 1 of the present invention shall be described hereafter.

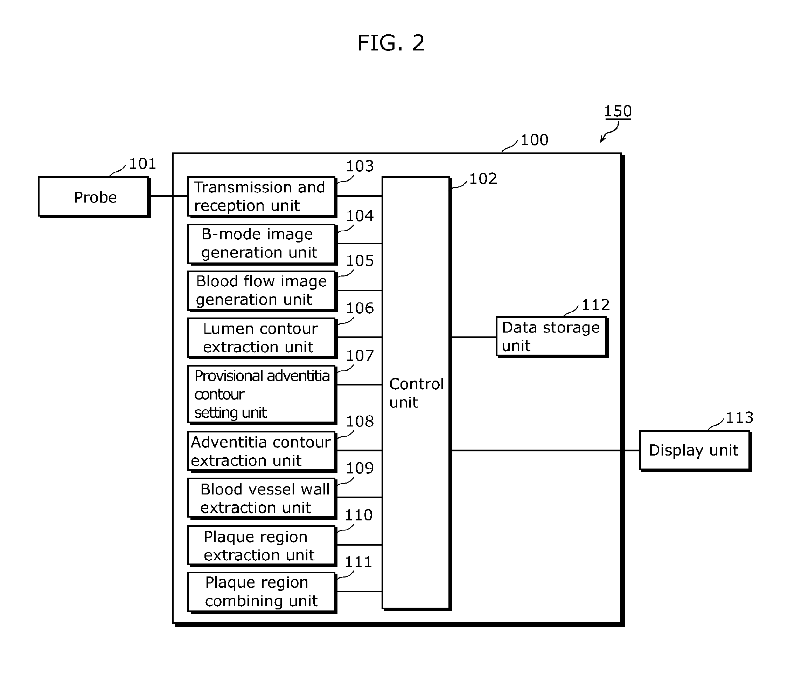

FIG. 2 is a block diagram showing a detailed configuration of the ultrasound diagnostic apparatus 150 according to Embodiment 1 of the present invention.

The ultrasound diagnostic apparatus 150 shown in FIG. 2 includes a ultrasound diagnostic apparatus main body 100, and a probe 101 and a display unit 113 which are connected to the ultrasound diagnostic apparatus main body 100. The ultrasound diagnostic apparatus main body 100 includes a control unit 102, a transmission and reception unit 103, the B-mode image generation unit 104, the blood flow image generation unit 105, the lumen contour extraction unit 106, the provisional adventitia contour setting unit 107, the adventitia contour extraction unit 108, a blood vessel wall extraction unit 109, a plaque region extraction unit 110, a plaque region combining unit 111, and a data storage unit 112.

The probe 101 is an ultrasound probe including ultrasound transducers which transmit ultrasound waves. The probe 101 transmits ultrasound waves according to the instructions of the transmission and reception unit 103. Furthermore, the probe 101 receives, as echo signals, the reflected ultrasound waves 201 (ultrasound reflected signals) from the subject. It should be noted that the probe 101 may be a probe in which the ultrasound transducers are arranged in a one-dimensional direction, or a two-dimensional array probe in which the ultrasound transducers are arranged in a matrix.

The control unit 102 controls the respective processing units included in the ultrasound diagnostic apparatus main body 100. Hereafter, although not specifically stated, the operations of the respective processing units are governed by the control unit 102, and the operations of the respective processing units are executed while the control unit 102 controls the operation timing, etc.

The transmission and reception unit 103 drives the ultrasound transducers of the probe 101 to cause the generation of ultrasound waves. Furthermore, the transmission and reception unit 103 receives the reflected ultrasound waves 201 received by the probe 101.

The B-mode image generation unit 104 generates a B-mode image 202 based on the reflected ultrasound waves 201 received by the transmission and reception unit 103. Specifically, the B-mode image generation unit 104 performs envelope detection after filtering processing is performed on the reflected ultrasound waves 201. In addition, the B-mode image generation unit 104 generates the B-mode image 202 by performing logarithmic conversion and gain adjustment on the detected signals.

The blood flow image generation unit 105 generates a blood flow image 203 based on the reflected ultrasound waves 201 received by the transmission and reception unit 103. Here, the blood flow image 203 is an image showing a region of the body in which blood flows. Specifically, the blood flow image generation unit 105 detects the speed of the blood flow within the blood vessel using the frequency change caused by the ultrasound waves being reflected off the blood flow. Then, the blood flow image generation unit 105 generates the blood flow image 203 by representing the detected speed of the blood flow as color data in an image. It should be noted that the color Doppler method or power Doppler method can be used, for example, as a method of imaging blood flow speed. Moreover, although the present embodiment describes the case where the blood flow image is represented by color data, the blood flow image 203 is not limited to color data and may be a black and white display.

The lumen contour extraction unit 106 extracts the lumen contour 402 of the blood vessel using the blood flow image 203 generated by the blood flow image generation unit 105.

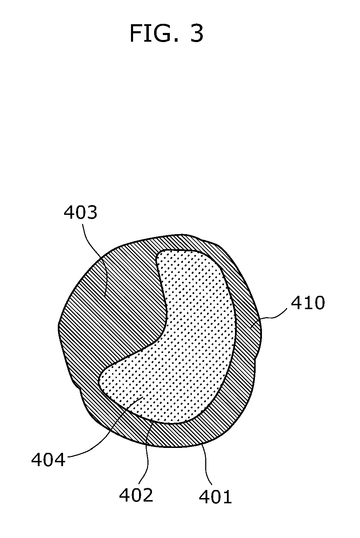

The shape of the blood vessel in the case where plaque is present in the blood vessel wall is described as follows. FIG. 3 is a cross-sectional view of a blood vessel in the case where plaque is present.

As shown in FIG. 3, the blood vessel includes a lumen 404 and a blood vessel wall 410. Furthermore, the lumen contour 402 is present in the periphery of the lumen 404. Specifically, the lumen contour 402 is the inner contour of the blood vessel and, stated differently, is the border between the lumen 404 and the blood vessel wall 410.

Furthermore, the adventitia contour 401 of the blood vessel is present in the outer side of the lumen contour 402. In other words, the adventitia contour 401 is the outer contour of the blood vessel.

Furthermore, a recessed portion is present in the lumen 404, and plaque 403 is present in the region of the blood vessel wall 410 in which the recessed portion is present.

Here, in the blood flow image 203, a part in which the blood flow is detected is displayed in color. Subsequently, the lumen contour extraction unit 106 extracts, using the blood flow image 203, the part in which color data is displayed, by extracting the region that is brighter than a predetermined brightness value. Here, the edge of the part in which the color data is displayed is extracted as the lumen contour 402 of the blood vessel. It should be noted that the lumen contour extraction unit 106 may extract the edge of the part in which the detected color data is displayed, itself, as the lumen contour 402, or extract the periphery of such edge as the lumen contour 402.

It should be noted that when all of parts in which color data is displayed are detected, there is a possibility of detecting even noise, and so on, which is not the blood vessel. In view of this, during or after extracting parts in which color data is displayed, the lumen contour extraction unit 106 extracts, as a blood flow region, a region having a surface area that is larger than a predetermined surface area.

It should be noted that the lumen contour extraction unit 106 may detect a region for which the brightness difference with a neighboring region is equal to or greater than a predetermined brightness difference, and specify that the border thereof is the lumen contour 402.

Furthermore, although the blood flow image generation unit 105 generates the blood flow image 203 here, an image need not necessarily be generated. Specifically, the blood flow image generation unit 105 may generate information (blood flow information) indicating a region in which blood flows, and the lumen contour extraction unit 106 may extract the lumen contour 402 using the blood flow information.

Furthermore, the lumen contour extraction unit 106 outputs lumen contour information indicating the extracted lumen contour 402 of the blood vessel to the control unit 102 and the blood vessel wall extraction unit 109. It should be noted that the lumen contour information may be outputted to the blood vessel wall extraction unit 109 by the provisional adventitia contour setting unit 107. Furthermore, as described later, the respective processing units may store lumen contour information, provisional adventitia contour information, and adventitia contour information in the data storage unit 112, and the respective processing units may obtain necessary information from the data storage unit 112.

Using the blood flow image 203, the provisional adventitia contour setting unit 107 sets the provisional adventitia contour 407 which is a temporary adventitia contour, based on the lumen contour 402 of the blood vessel extracted by the lumen contour extraction unit 106. Then, the provisional adventitia contour setting unit 107 transmits provisional adventitia contour information indicating the set provisional adventitia contour 407 to the adventitia contour extraction unit 108. The method of setting the provisional adventitia contour 407 shall be described in detail later.

After using the provisional adventitia contour 407 indicated by the provisional adventitia contour information in the B-mode image 202, the adventitia contour extraction unit 108 uses the provisional adventitia contour 407 as an initial contour to extract a more detailed adventitia contour 401 using the B-mode image 202. Then, the adventitia contour extraction unit 108 outputs adventitia contour information indicating the extracted adventitia contour 401 to the blood vessel wall extraction unit 109.

The blood vessel wall extraction unit 109 extracts a blood vessel wall region which is the region in which the blood vessel wall 410 is present, using the lumen contour 402 extracted by the lumen contour extraction unit 106 and the adventitia contour 401 extracted by the adventitia contour extraction unit 108. Specifically, the blood vessel wall extraction unit 109 extracts, as the blood vessel wall region, the region between the adventitia contour 401 and the lumen contour 402.

The plaque region extraction unit 110 extracts, using the blood vessel wall region extracted by the blood vessel wall extraction unit 109, a plaque region which is a region in which plaque 403 is present. Specifically, the plaque region extraction unit 110 extracts, as the plaque region, a region in which the thickness of the blood vessel wall 410 is greater than a predetermined threshold. The threshold used here is, for example, 1.1 mm. Furthermore, this threshold may be changed in accordance with the gender, age, and so on, of the subject.

It should be noted that, in the extraction of the plaque region, the plaque region extraction unit 110 may calculate the average value of the thickness of the blood vessel wall 410 for each of predetermined regions, compare the thickness of the blood vessel wall 410 in respective positions with the calculated average value, and extract, as the plaque region, positions in which the thickness of the blood vessel wall 410 is greater than the average value by a predetermined thickness or more.

Furthermore, the plaque region extraction unit 110 may calculate an overlapping part which overlaps with the blood vessel wall region, from among straight lines extending in a radial pattern from the center position of the adventitia contour 401, and extract, as the plaque region, an area having a length that is greater than the average value of lengths of overlapping parts in other areas.

It should be noted that, in the present embodiment, the presence of the plaque region is judged after the region between the adventitia contour 401 and the lumen contour 402 is extracted, as the blood vessel wall region, by the blood vessel wall extraction unit 109. However, after the lumen contour and the adventitia contour are extracted, the plaque region extraction unit 110 may calculate the distances between the respective contours and judge that an area is the plaque region when the calculated distance is equal to or greater than a predetermined distance. Stated differently, the ultrasound diagnostic apparatus 150 may extract the plaque region without extracting the blood vessel wall region.

The plaque region combining unit 111 combines the extracted plaque region with the B-mode image 202. Combining the plaque region with the B-mode image enables the examiner (observer) to visually recognize a region in which plaque is present. For example, displaying the region in which plaque is present using a high brightness value allows the examiner to easily recognize the plaque.

The data storage unit 112 holds the B-mode image 202 generated by the B-mode image generation unit 104, the blood flow image 203 generated by the blood flow image generation unit 105, the lumen contour information generated by the lumen contour extraction unit 106, the provisional adventitia contour information generated by the provisional adventitia contour setting unit 107, and the adventitia contour information generated by the adventitia contour extraction unit 108.

The display unit 113 is a display apparatus such as a liquid crystal display (LCD), and so on, and displays the B-mode image 202, the blood flow image 203, the data of the blood vessel wall 410, the data of the plaque 403, and so on. It should be noted that Embodiment 1 is characterized particularly by the contour extraction method for more accurately obtaining the lumen contour 402 and the adventitia contour 401. Therefore, whether or not to provide the ultrasound diagnostic apparatus 150 with the blood vessel wall extraction unit 109, the plaque region extraction unit 110, the plaque region combining unit 111, the data storage unit 112, and the display unit 113 is arbitrary.

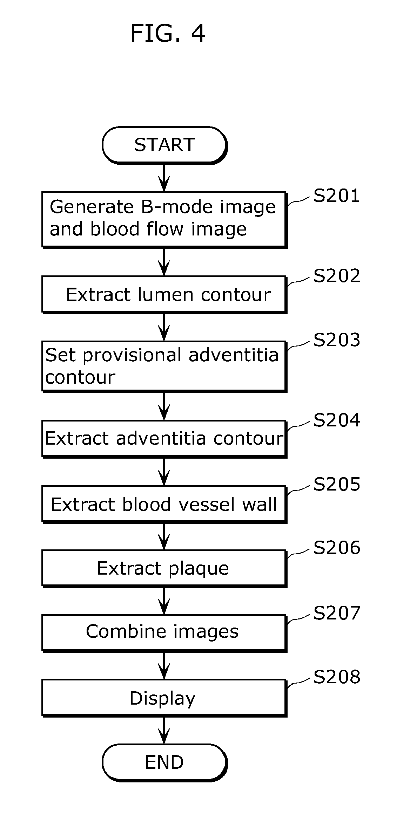

Next, the flow of the image processing for extracting the blood vessel wall 410, performed by the ultrasound diagnostic apparatus 150 shall be described with reference to the flowchart shown in FIG. 4.

First, in step S201, the B-mode image generation unit 104 generates a B-mode image 202, and the blood flow image generation unit 105 generates a blood flow image 203. Specifically, the transmission and reception unit 103 emits ultrasound waves into the subject via the probe 101 and receives reflected ultrasound waves 201 via the probe 101. The B-mode image generation unit 104 and the blood flow image generation unit 105 respectively generate the B-mode image 202 and the blood flow image 303 by processing the data received by the transmission and reception unit 103, and store the generated B-mode image 202 and blood flow image 303 in the data storage unit 112.

Next, in step S202, the lumen contour extraction unit 106 extracts a lumen contour 402 using the blood flow image 203. In the present invention, the lumen contour extraction unit 106 first extracts a blood flow region having an area that is greater than a predetermined value. There are cases where a small region which can be mistakenly judged as being a blood flow region is present in the blood flow image 203 during generation stage of the blood flow image 203. Therefore, by performing such processing, the carotid artery can be efficiently extracted from within the blood flow image 203. In addition, the lumen contour extraction unit 106 performs edge detection for the blood flow region, using the Sobel operator, and so on. Moreover, the lumen contour extraction unit 106 extracts 1 closed curve representing the edge shape of the blood flow region, after performing isolated line removal. The lumen contour extraction unit 106 stores information indicating such closed curve, as the lumen contour information, in the data storage unit 112.

In this manner, in the ultrasound diagnostic apparatus 150 according to the present embodiment, the lumen contour 402 of the blood vessel is extracted based on the blood flow image 203, instead of being extracted using the B-mode image 202. When the B-mode image 202 is used, there are cases where the contour of the blood vessel wall 410 is not displayed clearly and there are cases where accurate detection of the blood vessel wall 410 is difficult, depending on the running direction of the blood vessel. In contrast, by extracting the lumen contour 402 based on the blood flow image 203, the ultrasound diagnostic apparatus 150 is capable of tracing the inner wall of the blood vessel more accurately, regardless of the running direction of the blood vessel.

It should be noted that although in the present embodiment, a desired blood flow region is identified based on the surface area value, the blood flow region may be identified, taking into account temporal changes in blood flow images 203, such as pulsations. Furthermore, in such a case, it is preferable that the lumen contour 402 be extracted in a frame in which the surface area of the blood flow region is largest.

Next, the ultrasound diagnostic apparatus 150 calculates the adventitia contour 401 of the blood vessel using the lumen contour 402 extracted in step S202. First, problematic points in calculating the adventitia contour 401 shall be described.

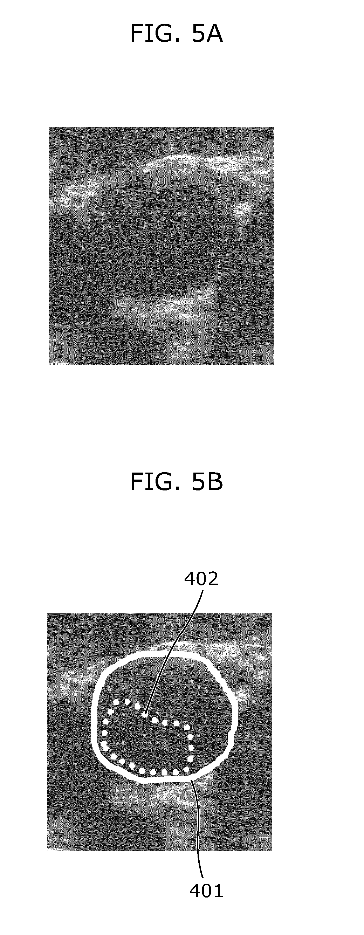

FIG. 5A is an image showing an example of the B-mode image 202 of the blood vessel. Furthermore, FIG. 5B is an image showing the lumen contour 402 and the adventitia contour 401 in the B-mode image 202 shown in FIG. 5A. As is clear from FIG. 5B, plaque 403 is present in the blood vessel wall 410, and, even when the shape of the lumen contour 402 is curved in a concave towards the inside of the blood vessel, in many cases a recess (or a protrusion) is not formed in the adventitia contour 401 and the adventitia contour 401 has an approximately circular shape.

The case of calculating the adventitia contour 401 by active contour searching, using the lumen contour 402 as an initial contour is described below.

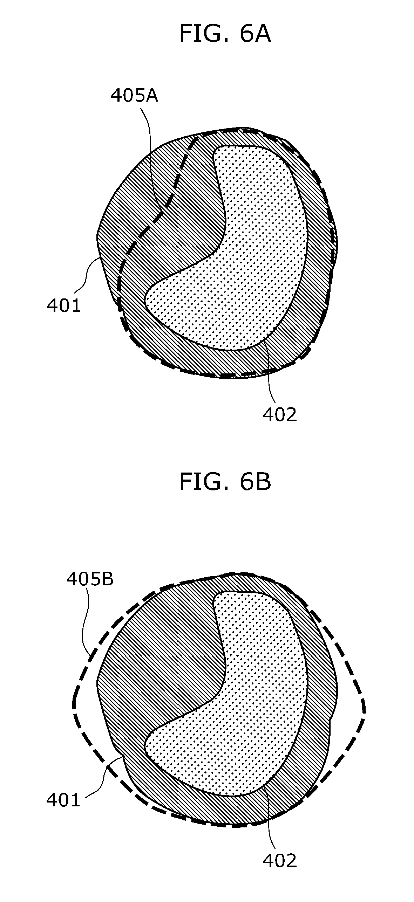

FIG. 6A is a diagram showing the shape of an adventitia contour 405a extracted when a normal energy function is set in active contour searching. Here, the adventitia contour 405A to be extracted is shown with a dotted line. When a normal energy function is set, the extracted adventitia contour 405A is influenced by the shape of the lumen contour 402 which is the initial contour. Accordingly, the contour of the part in which the plaque 403 is present becomes a shape that is sunken more than the actual contour.

As such, when the adventitia contour 401 of the blood vessel is extracted using the B-mode image 202, using the lumen contour 402 obtained in step S202 as the initial contour, it is necessary to set, in the active contour searching (for example, Snakes), an energy function for emphasizing outward searching.

Next, the case where an energy function for emphasizing outward searching is set shall be described. Here, the shape of an adventitia contour 405B that is extracted is shown in FIG. 6B.

As described earlier, there are cases where part of the lateral contour of the blood vessel is not clearly displayed in the B-mode image 202. For example, even in FIG. 5A, the vertical direction contour of the adventitia contour 401 is almost not displayed. As such, when active contour searching is performed, there is a possibility that, in a region in which the brightness difference is not clearly displayed as described above, the contour is not properly extracted and active contour searching is performed further outward. As such, when an energy function for emphasizing outward searching is set, the shape of the adventitia contour 405B that is extracted becomes a shape that projects outward as shown in FIG. 6B

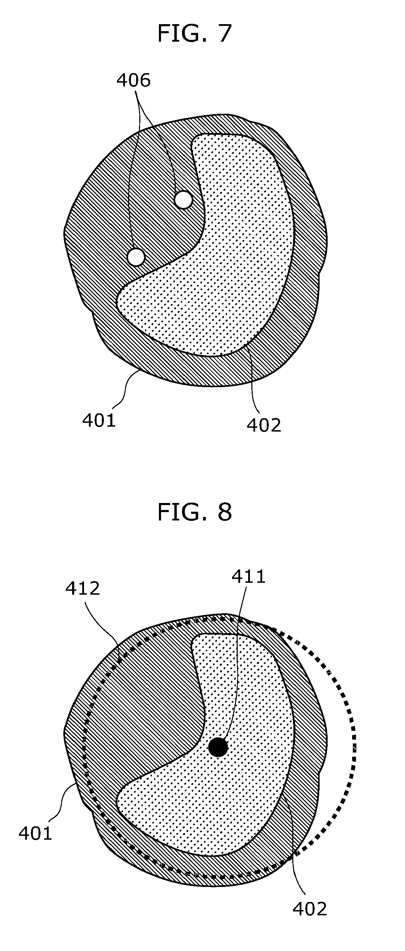

It should be noted that, even when the lumen contour 402 of the blood vessel has a distorted shape as shown in FIG. 5B, a method for extracting an approximately circular adventitia contour 401 is possible by making adjustments to the method for active contour searching. However, in a region in which the plaque 403 is present, the region between the intima wall and the adventitia wall of the blood vessel naturally widens. Specifically, as shown in FIG. 7, when the distance between the initial contour and the adventitia contour 401 that is actually found increases, the probability that high brightness regions 406 are present in the searched region increases. It should be noted that, here, a high brightness region 406 denotes noise. Specifically, noise caused by a calcified part or the scattering of ultrasound waves appears as a high brightness value. In addition, there is the problem that, since the active contour search converges in the high luminance regions 406, it is difficult to obtain the desired adventitia contour 401.

In view of this, in the ultrasound diagnostic apparatus 150 according to the present embodiment, the provisional adventitia contour 407 which is a temporary adventitia contour shape is set, from the blood flow image 203, using the lumen contour 402 obtained using the blood flow image 203. In addition, the ultrasound diagnostic apparatus 150 is characterized in calculating the adventitia contour 401 from a B-mode image 202, using the provisional adventitia contour 407 as an initial contour.

In step S203, the provisional adventitia contour setting unit 107 sets the provisional adventitia contour 407 using the blood flow image 203, based on the lumen contour 402 extracted in step S202. This process is described below.

As described above, it is preferable that the provisional adventitia contour 407, which is the initial contour during the searching of the adventitia contour 401, have an approximately circular shape that is close to the shape of the adventitia contour 401. Therefore, as shown in FIG. 8, the provisional adventitia contour setting unit 107 first calculates the coordinates of a provisional center point 411 of the lumen contour 402. For example, the provisional adventitia contour setting unit 107 calculates the coordinates of the provisional center point 411 by setting contour points at fixed intervals on the line of the lumen contour 402 and calculating an average value for the coordinate values of the contour points.

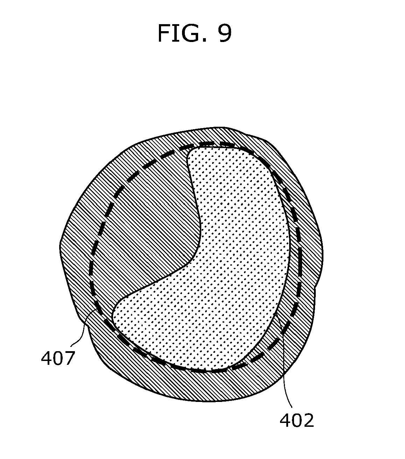

Next, the provisional adventitia contour setting unit 107 determines a circle 412 having the calculated provisional center point 411 as a center and which contains the lumen contour 402. For example, the circle 412 is a circle having a radius that is larger than the maximum value (maximum distance) of the length from the provisional center point 411 to the respective contour points on the lumen contour 402. Then, the provisional adventitia contour setting unit 107 determines, as the provisional adventitia contour 407, a contour that is obtained as a result of performing the active contour searching (Snakes, and so on) on the blood flow image 203 using the center 412 as an initial contour. Here, active contour searching is processing for extracting a contour by moving the contour points of the initial point by performing energy minimization. The provisional adventitia contour 407 is shown in FIG. 9.

Here, when the plaque 403 is present in the blood vessel wall 410, it is difficult to calculate the accurate center coordinates of the lumen contour 402. In response to this, in the present embodiment, the circle 412 in which the lumen contour 402 is contained is set, and then active contour searching is performed towards the inside using the circle 412 as the initial contour. With this, even when the coordinates of the provisional center point 411 is set displaced from the true coordinates, it is possible to obtain a provisional adventitia contour 407 having an approximately circular shape following the lumen contour 402.

The contour searching here is executed in order to obtain the general shape of the adventitia contour 401 which is approximately circular, and is not for the purpose of searching for the correct contour. As such, in the contour searching, it is preferable to perform a shape constraint such that the contour line does not become convex inward. This shape constraint, for example, in the case of applying the Snakes algorithm, can be implemented by increasing the weight of the inner deformation energy. Specifically, the provisional adventitia contour setting unit 107 performs a convergence processing by making the weight of the image energy smaller than the inner deformation energy in the energy minimization processing.

In the Snakes algorithm, the contour is determined to minimize energy $E_{snakes}$ defined in, for example, (Equation 1) to (Equation 3).

[Math 1]

$$E_{snakes} = \alpha E_{\mathrm{int}} + \beta E_{image}$$

(Equation 1)

[Math 2]

$$E_{\mathrm{int}} = \left(w_1 \left|\mathbf{v}_s\right|^2 + w_2 \left|\mathbf{v}_{ss}\right|^2\right)\Big/2$$

(Equation 2)

[Math 3]

$$E_{image} = -(G_\sigma * \nabla^2 I)^2$$

(Equation 3)

Here, $E_{\mathrm{int}}$ is the inner deformation energy of the contour line, $E_{image}$ is the image energy representing the conformity between the contour line and the image. v denotes a parameter expression of the contour line, $v_s$ denotes a first order differential of v, and $v_{ss}$ denotes a second order differential of v. $\alpha$, $\beta$, $w_1$, and $w_2$ denote constants indicating weight. G$\sigma$ denotes a Gaussian filter, $\nabla^2$ denotes a Laplacian filter, and I denotes the brightness value of the image. More specifically, the Snakes algorithm represents the contour line as contour points obtained by discretization of the contour line, and determines, for each contour point, a point such that energy $E_{snakes}$ is minimized. For example, by setting $\alpha$ = 0.8, and $\beta$ = 0.2, it is possible to search for the contour while maintaining the original approximately circular shape.

Furthermore, the adventitia contour 401 of the blood vessel cannot be extracted using the blood flow image 203. However, as described later, when finally extracting the adventitia contour 401 using the B-mode image 202, it is possible to extract the adventitia contour 401 precisely even when the adventitia contour 401 is not clear in the B-mode image 202, because the initial contour has an approximately circular shape that is close to the shape of the actual adventitia contour 401. In this manner, compared to the method of extracting the adventitia contour 401 using the lumen contour 402 as the initial contour, the method of extracting the adventitia contour 401 using the provisional adventitia contour 407 calculated by the above-described method allows more precise extraction of the adventitia contour 401.

In this manner, the provisional adventitia contour setting unit 107 sets a circular provisional adventitia contour 407 containing the lumen contour 402. Here, circular refers to a circle, an oval, and an approximately circular shape such as that described above.

Furthermore, the provisional adventitia contour 407 is set using the blood flow image 203. Unlike in the B-mode image 202, in the blood flow image 203, there is no high brightness region 406 such as noise, and so on, in the plaque region. As such, by setting the provisional adventitia contour 407 closer to the adventitia contour 401 than to the lumen contour 402, using the blood flow image 203, even when noise is present between the lumen contour 402 and the provisional adventitia contour 407 in the B-mode image 202, the provisional adventitia contour 407 can be set without being affected by the noise. Therefore, during adventitia contour searching, it is possible to reduce the probability of being affected by noise and converging to an erroneous contour.

In step S204, the adventitia contour extraction unit 108 extracts the adventitia contour 401 using the B-mode image 202, using, as the initial contour, the provisional adventitia contour 407 set in step S203. In the present embodiment, the adventitia contour extraction unit 108 extracts, as the adventitia contour 401, a contour obtained as a result of performing active contour searching using the provisional adventitia contour 407 as the initial contour.

For example, it is sufficient to apply the previously described Snakes algorithm. The adventitia contour extraction unit 108 sets the weights at this time as, for example, $\alpha$ = 0.5 and $\beta$ = 0.5. It should be noted that it is preferable that the adventitia contour extraction unit 108 change these weights depending on the state of depiction of the B-mode image 202. Specifically, the adventitia contour can be extracted precisely according to the state of depiction of the blood vessel contour by increasing the percentage of $\beta$ (increasing the percentage of the image energy) when the blood vessel contour is clear, and increasing the percentage of $\alpha$ (increasing the weight of the inner deformation energy) when the blood vessel contour is not clear. Specifically, the adventitia contour extraction unit 108 sets, to a second value, the weight of the image energy with respect to the inner deformation energy in the energy minimization processing when the clarity of the B-mode image 202 is a first value, and sets the weight to a fourth value larger than the second value when the clarity is a third value larger than the first value.

More preferably, the adventitia contour extraction unit 108 may change the weights according to the clarity on a contour point basis. Specifically, the adventitia contour extraction unit 108 increases the percentage of $\beta$ (increases the percentage of the image energy) for a contour point positioned at an anteroposterior walls of the blood vessel that is depicted clearly, and increases the percentage of $\alpha$ (increases the weight of the inner deformation energy) for a contour point positioned at the lateral walls which are unclearly depicted. With this, it is possible to precisely extract the adventitia contour 401 in accordance with the localized differences in depiction of the blood vessel wall 410. At this time, the adventitia contour extraction unit 108 may change the weights based on the position (for example, anteroposterior walls, lateral walls) of the contour point, and may change the weights based on the clarity of the depiction of the vicinity of the respective contour points. The clarity of the depiction may be measured, for example, by the difference between the maximum value and the minimum value of the brightness value in the vicinity of the contour point. It is considered that the wall depiction is clearer when the difference is bigger. It should be noted that the adventitia contour extraction unit 108 may use a different calculation method for the method of calculating clarity.

In step S205, the blood vessel wall extraction unit 109 extracts, as the blood vessel wall region, the region between the lumen contour 402 and the adventitia contour 401 obtained in steps S202 and S204, respectively. Then the plaque region extraction unit 110 extracts, as the plaque region, a region having a thickness that is greater than a predetermined value, from within the blood vessel wall region (S206). Subsequently, the plaque region combining unit 111 generates a combined image by overlapping information indicating such plaque region onto the B-mode image (S207), and the display unit 113 displays the generated combined image (S208).

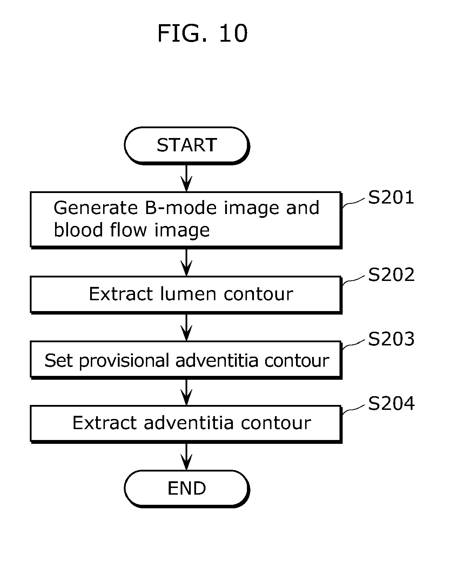

It should be noted that, as described earlier, the adoption of the blood vessel wall extraction step S205, the plaque extraction step S206, the image combining step S207, and the display step S208 is arbitrary. In other words, as shown in FIG. 10, the ultrasound diagnostic apparatus 150 may perform just the processing in steps S201 to S204.

Furthermore, in step S207, the plaque region combining unit 111 may generate the combined image by overlapping at least one of (i) the blood vessel wall region and (ii) the lumen contour 402 and the adventitia contour 401 onto the B-mode image, in addition to the plaque region.

Furthermore, the ultrasound diagnostic apparatus 150 may generate the combined image by overlapping the information indicating the blood vessel wall region onto the B-mode image, and displaying the combined image, without performing the processing in step S206. Furthermore, the ultrasound diagnostic apparatus 150 may generate the combined image by overlapping information indicating the lumen contour 402 and the adventitia contour 401 onto the B-mode image, and displaying the combined image, without performing the processing in steps S205 and S206.

In the manner described above, the ultrasound diagnostic apparatus 150 according to Embodiment 1 of the present invention calculates the shape of the blood vessel intima from the shape of the blood flow region, and extracts the contour of the adventitia using the shape information of the intima. With this, the ultrasound diagnostic apparatus 150 is able to obtain, more stably and accurately, the position information of the intima and adventitia of the blood vessel. As a result, the ultrasound diagnostic apparatus 150 is capable of more accurately judging the presence of plaque.

It should be noted that although the case where the ultrasound diagnostic apparatus 150 displays a combined image obtained by superimposing the plaque region onto the B-mode image 202, the ultrasound diagnostic apparatus may represent the plaque region as volume data, by performing the above-described processing on respective cross-sectional images having different scanning positions and reconfiguring the extracted plaque regions as three-dimensional data.

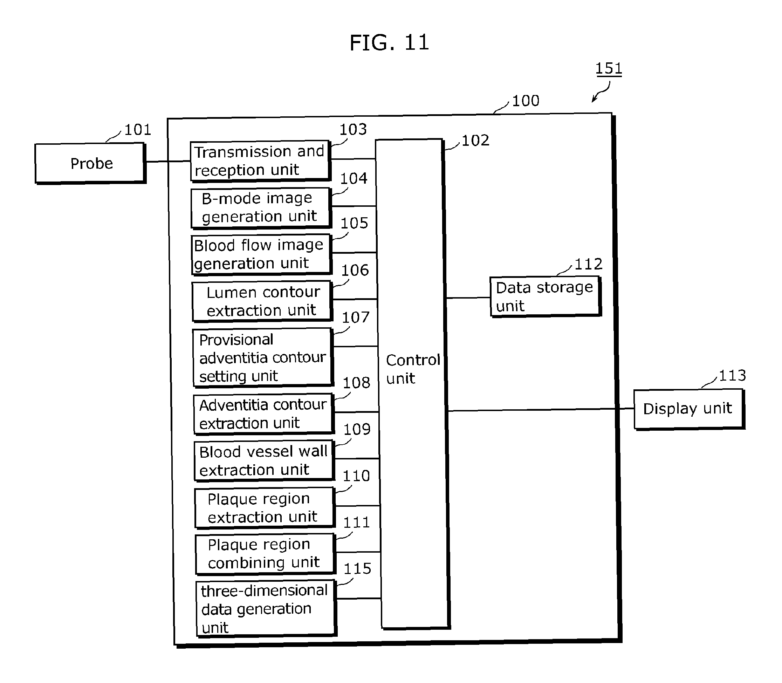

FIG. 11 is a diagram showing a configuration of a ultrasound diagnostic apparatus 151 in the above case. The ultrasound diagnostic apparatus 151 shown in FIG. 11 further includes a three-dimensional data generation unit 115 in addition to the configuration shown in FIG. 2.

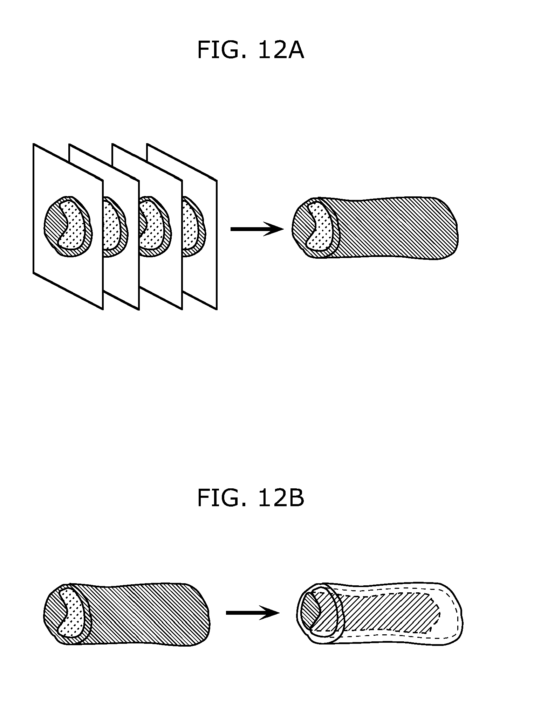

First, the ultrasound diagnostic apparatus 151 performs, on the respective cross-sectional images, processing for extracting the above-described blood vessel wall region and plaque region. The three-dimensional data generation unit 115 generates three-dimensional data indicating the blood vessel wall region and the plaque region, based on the information on the blood vessel wall regions and plaque regions extracted from the cross-sectional images. For example, the three-dimensional data generation unit 115 generates three-dimensional data of the blood vessel from the cross-sectional images from which the lumen contour 402 and adventitia contour 401 or the blood vessel wall region are extracted, as shown in FIG. 12A. In addition, the three-dimensional data generation unit 115 generates three-dimensional data for the highlighted-displaying of the plaque region in the three-dimensional data, using the plaque region extracted in the cross-sectional images, as shown in FIG. 12B. Then, the display unit 113 displays the three-dimensional data generated by the three-dimensional data generation unit 115.

It should be noted that the ultrasound diagnostic apparatus 150 may display the three-dimensional data shown in FIG. 12A, without performing the processing in step S206.

[Embodiment 2]

In Embodiment 2 of the present invention, a modification of the above-described Embodiment 1 shall be described.

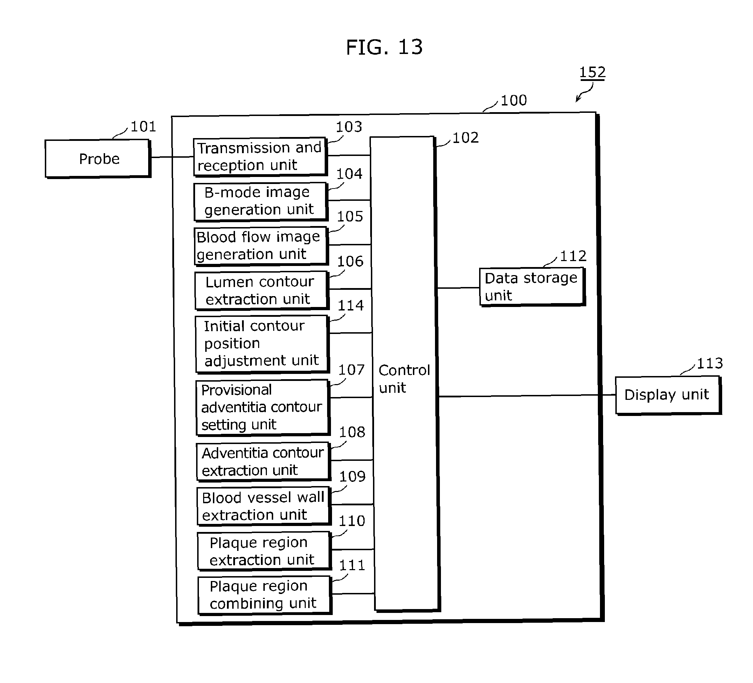

FIG. 13 is a block diagram showing a configuration of the ultrasound diagnostic apparatus 152 according to Embodiment 2 of the present invention. It should be noted that, in FIG. 13, constituent elements that are the same as those in FIG. 2 use the same reference numerals, and their description shall not be repeated.

The ultrasound diagnostic apparatus 152 shown in FIG. 13 includes an initial contour position adjustment unit 114 in addition to the configuration of the ultrasound diagnostic apparatus 150 shown in FIG. 2.

The initial contour position adjustment unit 114 adjusts the position of the provisional adventitia contour 407, based on the B-mode image 202. Specifically, due to the difference in the obtainment timing of the blood flow image 203 and the B-mode image 202, there are instances where the position of the blood vessel in the respective images is different. Since the provisional adventitia contour 407 can be obtained based on the blood flow image 203, it is necessary to adjust such provisional adventitia contour 407 to the position of the adventitia contour 401 in the B-mode image 202. In the present embodiment, the initial contour position adjustment unit 114 calculates, from brightness data of the B-mode image 202, the approximate center coordinates of the blood vessel image in the B-mode image 202, and moves the provisional adventitia contour 407 such that the center of the provisional adventitia contour 407 approaches the calculated approximate center coordinates. Specifically, the initial contour position adjustment unit 114 adjusts the position of the provisional adventitia contour 407 to match the center of the provisional adventitia contour 407 to the calculated approximate center coordinates. Since the blood vessel lumen is generally depicted with low brightness in the B-mode image 202, the initial contour position adjustment unit 114 searches for a low-brightness region in the vicinity of the original position of the provisional adventitia contour 407, and adopts the center of the low-brightness region as the center position of the blood vessel image.

According to the present configuration, the ultrasound diagnostic apparatus 152 according to Embodiment 2 of the present invention is capable of stably obtaining a correct adventitia contour 401 even when there is a misalignment of images between the B-mode image 202 and the blood flow image 203. In this manner, the ultrasound diagnostic apparatus 152 is capable of more accurately judging the presence of plaque.

Although the ultrasound diagnostic apparatuses according to the embodiments of the present invention have been described up to this point, the present invention is not limited to these embodiments.

For example, part or all of the processing units included in the ultrasound diagnostic apparatus main body 100 may be included in the probe 101.

Furthermore, although the foregoing description is carried out exemplifying the case of using, as the B-mode image and the blood flow image, what is called a short axis view which shows a blood vessel cross-section which is perpendicular to the direction in which the blood vessel extends, the present invention can also be applied to the case of using what is called a long axis view which is a cross-sectional view of the blood vessel, which is parallel to the direction in which the blood vessel extends. In such a case, the provisional adventitia contour setting unit 107 determines a quadrangular provisional adventitia contour containing the lumen contour. Here, quadrangular refers to a rectangle, a parallelogram, and an approximately rectangular shape.

Furthermore, each of the processing units included in the ultrasound diagnostic apparatus according to the above-described embodiments is typically implemented as an LSI which is an integrated circuit. These processing units may be individually configured as single chips or may be configured so that a part or all of the processing units are included in a single chip.

Furthermore, the method of circuit integration is not limited to LSIs, and implementation through a dedicated circuit or a general-purpose processor is also possible. A Field Programmable Gate Array (FPGA) which allows programming after LSI manufacturing or a reconfigurable processor which allows reconfiguration of the connections and settings of the circuit cells inside the LSI may also be used.

Furthermore, part or all of the functions of the ultrasound diagnostic apparatus, according to the embodiments of the present invention may be implemented through the execution of a program by a processor such as a CPU.

In addition, the present invention may be the aforementioned program or a non-transitory computer-readable recording medium on which such program is recorded. Furthermore, it should be obvious that the program can also be distributed via a transmission medium such as the Internet.

Furthermore, at least part of the functions of the ultrasound diagnostic apparatuses according to Embodiments 1, 2, and their modifications may be combined.

Furthermore, all the numerical figures used above are given as examples to describe the present invention in specific terms, and thus the present invention is not limited by such illustrative numerical figures.

Furthermore, the separation of the function blocks in the block diagrams is merely an example, and plural function blocks may be implemented as a single function block, a single function block may be separated into plural function blocks, or part of functions of a function block may be transferred to another function block. Furthermore, the functions of function blocks having similar functions may be processed, in parallel or by time-sharing, by a single hardware or software.

Furthermore, the sequence in which the above-described steps are executed is given as an example to describe the present invention in specific terms, and thus other sequences are possible. Furthermore, part of the above-described steps may be executed simultaneously (in parallel) with another step.

In addition, as long as they do not depart from the essence of the present invention, various modifications obtainable through modifications to the respective embodiments that may be conceived by a person of ordinary skill in the art are intended to be included in the present invention.

[Industrial Applicability]

The present invention is applicable to ultrasound diagnostic apparatuses. Furthermore, the present invention is useful in the diagnosis of arteriosclerosis using an ultrasound diagnostic apparatus. In addition, the present invention can also be used in measuring the degree of stenosis using an ultrasound diagnostic apparatus.

[Reference Signs List]

| | |
|---|---|
| 100 | Ultrasound diagnostic apparatus main body |
| 101 | Probe |
| 102 | Control unit |
| 103 | Transmission and reception unit |
| 104 | B-mode image generation unit |
| 105 | Blood flow image generation unit |
| 106 | Lumen contour extraction unit |
| 107 | Provisional adventitia contour setting unit |
| 108 | Adventitia contour extraction unit |
| 109 | Blood vessel wall extraction unit |
| 110 | Plaque region extraction unit |
| 111 | Plaque region combining unit |
| 112 | Data storage unit |
| 113 | Display unit |
| 114 | Initial contour position adjustment unit |
| 115 | Three-dimensional data generation unit |
| 150, 151, 152 | Ultrasound diagnostic apparatus |
| 201 | Reflected ultrasound waves |
| 202 | B-mode image 202 |
| 203 | Blood flow image |
| 401 | Adventitia contour |
| 402 | Lumen contour |
| 403 | Plaque |
| 404 | Lumen |
| 405A, 405B | Extracted adventitia contour |
| 406 | High brightness region (noise) |
| 407 | Provisional adventitia contour |
| 410 | Blood vessel wall |
| 411 | Provisional center point |
| 412 | Circle |

**Claims**

1. An ultrasound diagnostic apparatus which observes an inside of a body of a subject based on ultrasound waves reflected from the inside of the body and obtained using an ultrasound probe, said ultrasound diagnostic apparatus comprising:

   a B-mode image generation unit configured to generate a B-mode image, based on the reflected ultrasound waves;
   a blood flow information generation unit configured to generate, based on the reflected ultrasound waves, blood flow information representing a region of the body in which blood flows;

a lumen contour extraction unit configured to extract a lumen contour of a blood vessel, based on the blood flow information;

a provisional adventitia contour setting unit configured to set a provisional adventitia contour containing the lumen contour; and

an adventitia contour extraction unit configured to extract an adventitia contour of the blood vessel, using the B-mode image, and using the provisional adventitia contour as a first initial contour.

2. The ultrasound diagnostic apparatus according to Claim 1, further comprising:

a blood vessel wall extraction unit configured to extract, using the lumen contour and the adventitia contour, a blood vessel wall region in which a blood vessel wall is present; and

a plaque region extraction unit configured to extract, using the blood vessel wall region, a plaque region in which plaque is present.

3. The ultrasound diagnostic apparatus according to Claim 2, further comprising:

a three-dimensional data generation unit configured to generate three-dimensional data representing the blood vessel wall region and the plaque region; and

a display unit configured to display the three-dimensional data.

4. The ultrasound diagnostic apparatus according to any one of Claims 1 to 3,
wherein said provisional adventitia contour setting unit is configured to set the provisional adventitia contour having a circular shape.

5. The ultrasound diagnostic apparatus according to Claim 4, wherein said provisional adventitia contour setting unit is configured to:

set a circle having a center point of the lumen contour as a center and a radius that is greater than a maximum distance from the center point to the lumen contour, and

perform, on the blood flow information and using the set circle as a second initial contour, first active contour searching to search for a contour such that a sum of an inner deformation energy of a contour line and an image energy is smallest, and set the contour obtained through the search as the provisional adventitia contour, the image energy representing conformity between the contour line and an image.

6. The ultrasound diagnostic apparatus according to Claim 5,
wherein said provisional adventitia contour setting unit is configured to perform convergence processing by making a weight of the image energy smaller than a weight of the inner deformation energy in the first active contour searching.

7. The ultrasound diagnostic apparatus according to any one of Claims 1 to 6,
wherein said adventitia contour extraction unit is configured to perform, on the B-mode image and using the provisional adventitia contour as the first initial contour, second active contour searching to search for a contour such that a sum of an inner deformation energy of a contour line and an image energy is smallest, and set the contour obtained through the search as the provisional adventitia contour, the image energy representing conformity between the contour line and an image.

8. The ultrasound diagnostic apparatus according to Claim 7,
wherein said provisional adventitia contour setting unit is configured to set a weight of the image energy with respect to the inner deformation energy to a second value when clarity of the B-mode image is a first value, and to set the weight to a fourth value which is larger than the second value when the clarity is a third value larger than the first value, in the second active contour searching.

9. The ultrasound diagnostic apparatus according to Claim 8,
wherein said provisional adventitia contour setting unit is configured to change the weight in accordance with the clarity, for each of contour points in the B-mode image.

10. The ultrasound diagnostic apparatus according to any one of Claims 1 to 9,
wherein said blood flow information generation unit is configured to generate the blood flow information, using a color Doppler method.

**11.** The ultrasound diagnostic apparatus according to any one of Claims 1 to 10,
wherein said provisional adventitia extraction unit includes an initial contour position adjustment unit, and
said initial contour position adjustment unit is configured to:

calculate a center point of a blood vessel region, using the B-mode image; and
move the provisional adventitia contour so that a center point of the provisional adventitia contour approaches
the center point of the blood vessel region.

**12.** An ultrasound diagnostic method of observing an inside of a body of a subject based on ultrasound waves reflected
from the inside of the body and obtained using an ultrasound probe, said ultrasound diagnostic method comprising:

generating a B-mode image, based on the reflected ultrasound waves;
generating blood flow information representing a region in which blood flows, based on the reflected ultrasound
waves;
extracting a lumen contour of a blood vessel, based on the blood flow information;
setting a provisional adventitia contour containing the lumen contour; and
extracting an adventitia contour of the blood vessel, using the B-mode image, and using the provisional adventitia
contour as a first initial contour.

FIG. 1

EP 2 626 009 A1

## FIG. 2

Probe (101) → Transmission and reception unit (103)

- Transmission and reception unit (103)
- B-mode image generation unit (104)
- Blood flow image generation unit (105)
- Lumen contour extraction unit (106)
- Provisional adventitia contour setting unit (107)
- Adventitia contour extraction unit (108)
- Blood vessel wall extraction unit (109)
- Plaque region extraction unit (110)
- Plaque region combining unit (111)

Control unit (102)

Data storage unit (112)

Display unit (113)

100 / 150

EP 2 626 009 A1

# FIG. 3

# FIG. 4

START

Generate B-mode image and blood flow image — S201

Extract lumen contour — S202

Set provisional adventitia contour — S203

Extract adventitia contour — S204

Extract blood vessel wall — S205

Extract plaque — S206

Combine images — S207

Display — S208

END

## FIG. 5A

## FIG. 5B

# FIG. 6A

405A

401

402

# FIG. 6B

405B

401

402

EP 2 626 009 A1

## FIG. 7

## FIG. 8

FIG. 9

# FIG. 10

```
        ┌─────────────┐
        │    START    │
        └─────────────┘
               │
               ▼
  ┌───────────────────────────┐  ╱ S201
  │ Generate B-mode image and │
  │ blood flow image          │
  └───────────────────────────┘
               │
               ▼
  ┌───────────────────────────┐  ╱ S202
  │   Extract lumen contour   │
  └───────────────────────────┘
               │
               ▼
  ┌───────────────────────────┐  ╱ S203
  │ Set provisional adventitia contour │
  └───────────────────────────┘
               │
               ▼
  ┌───────────────────────────┐  ╱ S204
  │  Extract adventitia contour │
  └───────────────────────────┘
               │
               ▼
        ┌─────────────┐
        │     END     │
        └─────────────┘
```

EP 2 626 009 A1

## FIG. 11

151

100

Probe 101 → Transmission and reception unit 103

B-mode image generation unit 104

Blood flow image generation unit 105

Lumen contour extraction unit 106

Provisional adventitia contour setting unit 107

Adventitia contour extraction unit 108

Blood vessel wall extraction unit 109

Plaque region extraction unit 110

Plaque region combining unit 111

three-dimensional data generation unit 115

Control unit 102

Data storage unit 112

Display unit 113

FIG. 12A

FIG. 12B

FIG. 13

Probe 101

152
100
102

Transmission and reception unit 103
B-mode image generation unit 104
Blood flow image generation unit 105
Lumen contour extraction unit 106
Initial contour position adjustment unit 114
Provisional adventitia contour setting unit 107
Adventitia contour extraction unit 108
Blood vessel wall extraction unit 109
Plaque region extraction unit 110
Plaque region combining unit 111

Control unit

Data storage unit 112

Display unit 113

# EP 2 626 009 A1

<table>
<tr><td colspan="2" align="center"><strong>INTERNATIONAL SEARCH REPORT</strong></td><td colspan="2">International application No.<br>PCT/JP2011/005568</td></tr>
</table>

**A. CLASSIFICATION OF SUBJECT MATTER**
*A61B8/08*(2006.01)i, *A61B8/06*(2006.01)i

According to International Patent Classification (IPC) or to both national classification and IPC

**B. FIELDS SEARCHED**

Minimum documentation searched (classification system followed by classification symbols)
A61B8/08, A61B8/06

Documentation searched other than minimum documentation to the extent that such documents are included in the fields searched
Jitsuyo Shinan Koho            1922-1996   Jitsuyo Shinan Toroku Koho    1996-2011
Kokai Jitsuyo Shinan Koho      1971-2011   Toroku Jitsuyo Shinan Koho    1994-2011

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br>A | JP 2009-28096 A   (Yamaguchi University),<br>12 February 2009 (12.02.2009),<br>paragraphs [0010] to [0015]; fig. 8, 13<br>(Family: none) | 1-4,7-10<br>5-6,11 |
| Y<br>A | WO 2005/087111 A1  (Hitachi Medical Corp.),<br>22 September 2005 (22.09.2005),<br>paragraphs [0061] to [0078]; fig. 13, 14<br>& US 2007/0149877 A1     & EP 1728471 A1<br>& CN 1921802 A | 1-4,7-10<br>5-6,11 |
| Y<br>A | WO 2007/34738 A1  (Panasonic Corp.),<br>29 March 2007 (29.03.2007),<br>paragraph [0046]<br>& US 2009/0143675 A1     & EP 1927317 A1 | 1-4,7-10<br>5-6,11 |

[X] Further documents are listed in the continuation of Box C.      [ ] See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>  08 December, 2011 (08.12.11) | Date of mailing of the international search report<br>  20 December, 2011 (20.12.11) |
| Name and mailing address of the ISA/<br>  Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2011/005568 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 2005-28123 A (Saraya Co., Ltd., Media Cross Kabushiki Kaisha), 03 February 2005 (03.02.2005), paragraphs [0180] to [0184]; fig. 31, 32 (Family: none) | 4 |
| Y | JP 2000-331143 A (Mitsubishi Electric Corp.), 30 November 2000 (30.11.2000), paragraphs [0050] to [0052] (Family: none) | 7-9 |
| A | JP 2000-271117 A (Aloka Co., Ltd.), 03 October 2000 (03.10.2000), fig. 3, 6 (Family: none) | 1-11 |

Form PCT/ISA/210 (continuation of second sheet) (July 2009)

| INTERNATIONAL SEARCH REPORT | International application No. |
|---|---|
| | PCT/JP2011/005568 |

**Box No. II      Observations where certain claims were found unsearchable (Continuation of item 2 of first sheet)**

This international search report has not been established in respect of certain claims under Article 17(2)(a) for the following reasons:

1. ☒   Claims Nos.: 12

     because they relate to subject matter not required to be searched by this Authority, namely:

     The invention in claim 12 is considered to be concerned with method which is executed in a state such that an ultrasonic probe inserted into a human body is maintained, and therefore pertains to "method for treatment of a human body by surgical operation", (continued to extra sheet)

2. ☐   Claims Nos.:

     because they relate to parts of the international application that do not comply with the prescribed requirements to such an extent that no meaningful international search can be carried out, specifically:

3. ☐   Claims Nos.:

     because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐   As all required additional search fees were timely paid by the applicant, this international search report covers all searchable claims.

2. ☐   As all searchable claims could be searched without effort justifying additional fees, this Authority did not invite payment of additional fees.

3. ☐   As only some of the required additional search fees were timely paid by the applicant, this international search report covers only those claims for which fees were paid, specifically claims Nos.:

4. ☐   No required additional search fees were timely paid by the applicant. Consequently, this international search report is restricted to the invention first mentioned in the claims; it is covered by claims Nos.:

**Remark on Protest**      ☐   The additional search fees were accompanied by the applicant's protest and, where applicable, the payment of a protest fee.

     ☐   The additional search fees were accompanied by the applicant's protest but the applicable protest fee was not paid within the time limit specified in the invitation.

     ☐   No protest accompanied the payment of additional search fees.

Form PCT/ISA/210 (continuation of first sheet (2)) (July 2009)

**INTERNATIONAL SEARCH REPORT**

International application No.

PCT/JP2011/005568

Continuation of Box No.II-1 of continuation of first sheet(2)

and thus relates to a subject matter on which an international search is not required to be carried out under the provision of PCT Rule 39.1(iv).

Form PCT/ISA/210 (extra sheet) (July 2009)

31

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3468869 B

**Non-patent literature cited in the description**

- **AINSWORTH CD ; BLAKE CC ; TAMAYO A ; BELETSKY V ; FENSTER A ; SPENCE JD.** 3D ultrasound measurement of change in carotid plaque volume: a tool for rapid evaluation of new therapies. *Stroke,* September 2005, vol. 36 (9), 1904-1909

- **J.C.R. SEABRA ; L.M. PEDRO ; J.F. E FERNANDES ; J.M. SANCHES.** A 3-D Ultrasound based Framework to Characterize the Echo Morphology of Carotid Plaques. *IEEE Trans. Biomed. Eng.,* 2009, vol. 56, 1442